# EUROPEAN PATENT APPLICATION

(11) **EP 4 328 928 A1**
(43) Date of publication of application: **28.02.2024**
(21) Application number: 23193393.8
(22) Date of filing: 25.08.2023
(51) Int. Cl.: G16H 20/70

(54) **METHOD AND DEVICE FOR CONTROLLING IMPROVED COGNITIVE FUNCTION TRAINING APP**

(30) Priority: 25.08.2022 KR 20220106636; 03.04.2023 KR 20230043674
(71) Applicant: Emocog Co., Ltd., Seoul 08813 (KR)
(72) Inventor: NOH, Yoo Hun, 06346 Seoul (KR)
(74) Representative: Zacco Sweden AB

(57) **Abstract**

Provided is a method of controlling an improved cognitive function training app, the method including determining whether the cognitive function training app has been initiated by an input from a user to a terminal, optimizing cognitive function training provided by the cognitive function training app to be appropriate for the user, based on a result of the determining of whether the cognitive function training app has been initiated, and information of the user of the terminal, and based on the cognitive function training app being initiated or resumed by an input from the user, providing the optimized cognitive function training through the terminal.

## Description

### BACKGROUND

### 1. Field

The present disclosure relates to a method of controlling an application installed in a terminal, and more particularly, to a method of controlling an application for training a cognitive function, and a device for implementing the method.

### 2. Description of the Related Art

It is evaluated that the Republic of Korea has already entered an aging society. As the aging society progresses, the number of dementia patients rapidly increases, and social costs for treating and caring for the dementia patients also gradually increase.

It has already been proven that training for stimulating areas of the brain responsible for cognitive functions is effective in preventing dementia and improving symptoms of dementia that is already occurred, and that, through several papers, by training a patient with a mild cognitive impairment corresponding to a prior stage of dementia to improve his/her cognitive function, the cognitive function significantly improves compared to a patient who already has dementia.

### SUMMARY

Provided are a method of controlling a cognitive function training app, and a device for implementing the method.

Additional aspects will be set forth in part in the description which follows and, in part, will be apparent from the description, or may be learned by practice of the presented embodiments of the disclosure.

According to an aspect of an embodiment, a method of controlling an improved cognitive function training app includes determining whether the cognitive function training app has been initiated by an input from a user to a terminal, optimizing cognitive function training provided by the cognitive function training app to be appropriate for the user, based on a result of the determining of whether the cognitive function training app has been initiated, and information of the user of the terminal, and based on the cognitive function training app being initiated or resumed by an input from the user, providing the optimized cognitive function training through the terminal.

In the method, the information of the user may be information that is previously collected by a sensor of the terminal.

In the method, the information of the user may be information about a biosignal-based circadian rhythm of the user collected by the sensor of the user terminal, and a schedule of the user.

In the method, the information of the user may be at least one of a sleeping time of the user, the number of housemates of the user, an outdoor activity level of the user, and a depression level of the user, which are input after the cognitive function training app is first executed.

In the method, the optimizing may include visually or aurally outputting a training time range of day that is most appropriate for the user.

In the method, the information of the user may be external information about a day on which the cognitive function training app provides the cognitive function training, and personal information of the user on the day.

In the method, the optimizing may include, after determining that the cognitive function training app has been initiated, optimizing the cognitive function training in real time by considering collected inputs from the user.

In the method, based on the inputs from the user being greater than a preset level, the cognitive function training may be stopped for a preset time period, or a training speed of the cognitive function training may be changed.

In the method, based on the inputs from the user being less than or equal to a preset level, the cognitive function training may be stopped for a preset time period, or a training speed of the cognitive function training may be changed.

In the method, inputs irrelevant to the user may be collected in addition to the inputs from the user, and based on the collected inputs being greater than a preset level, the cognitive function training may be stopped.

In the method, the providing of the optimized cognitive function training through the terminal may include stopping the cognitive function training until the collected inputs fall below the certain level.

In the method, based on a new input from the user satisfying a preset condition, at least one of visual guide information and auditory guide information may be output through the terminal.

In the method, a fatigue level of the user may be calculated based on the input from the user, and a progress speed of the optimized cognitive function training may be adjusted based on the calculated fatigue level.

According to an aspect of another embodiment, an apparatus for controlling an improved cognitive function training app includes an initiation determination unit configured to determine whether the cognitive function training app has been initiated by an input from a user to a terminal, a training optimization unit configured to optimize cognitive function training provided by the cognitive function training app to be appropriate for the user, based on a result of the determining of whether the cognitive function training app has been initiated, and information of the user of the terminal, and a training provision unit configured to, based on the cognitive function training app being initiated or resumed by an input from the user, provide the optimized cognitive function training through the terminal.

According to an aspect of another embodiment, there may be provided a computer program stored in a computer-readable storage device for executing the method.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a diagram schematically illustrating an entire system for implementing the present disclosure;
FIG. 2 is a block diagram illustrating a training app as a logical device, according to an embodiment of the present disclosure;
FIG. 3 is a block diagram illustrating an example of a processing unit subdivided by function;
FIG. 4 is a flowchart illustrating an example of an operation process of submodules of the processing unit of FIG. 3;
FIG. 5 is a diagram for describing a method of controlling an improved cognitive function training app, according to an embodiment of the present disclosure;
FIG. 6 is a diagram for describing an embodiment in which cognitive function training is optimized as the absence of a user is detected;
FIGS. 7A and 7B are diagrams schematically illustrating examples in which cognitive function training is optimized in a situation in which a user's voice is normally input;
FIG. 8 is a diagram exemplarily illustrating a situation in which cognitive function training is stopped;
FIG. 9 is a diagram schematically illustrating an example of outputting a training time range most appropriate for a user; and
FIG. 10 is a flowchart illustrating an example of a method of controlling a cognitive function training app of FIGS. 5 to 9.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

As the present disclosure allows for various changes and numerous embodiments, particular embodiments will be illustrated in the drawings and described in detail. The effects and features of the present disclosure and methods of achieving them will become clear with reference to the embodiments described in detail below with the drawings. However, the present disclosure is not limited to the embodiments disclosed below, and may be implemented in various forms.

Hereinafter, embodiments of the present disclosure will be described in detail with reference to the accompanying drawings, and the same or corresponding components will be denoted by the same reference numerals when described with reference to the accompanying drawings, and thus, their descriptions that are already provided will be omitted.

In the following embodiments, terms such as "first," "second," etc., are used only to distinguish one component from another, and such components must not be limited by these terms.

In the following embodiments, the singular expression also includes the plural meaning as long as it is not inconsistent with the context.

In the following embodiments, the terms "comprises," "includes," "has", and the like used herein specify the presence of stated features or components, but do not preclude the presence or addition of one or more other features or components.

When a certain embodiment may be differently implemented, particular operations may be performed differently from the sequence described herein. For example, two processes, which are successively described herein, may be substantially simultaneously performed, or may be performed in a process sequence opposite to a described process sequence.

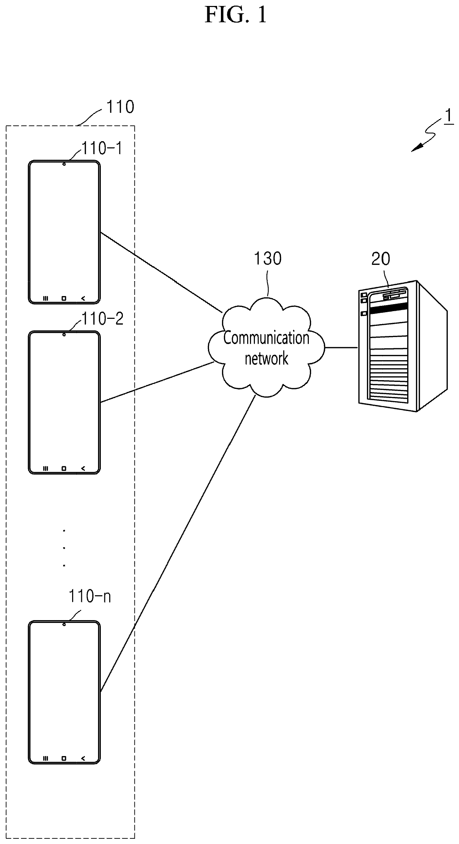

FIG. 1 is a diagram schematically illustrating an entire system for implementing the present disclosure.

Referring to FIG. 1, an entire system 1 according to the present disclosure has a structure in which a user terminal group 110 and a management server 20 are connected to each other through a communication network 130.

The user terminal group 110 may include one or more user terminals. For example, the number of user terminals included in the user terminal group 110 may be one or n as illustrated in FIG. 1. In FIG. 1, n may be an integer greater than or equal to 1.

Each user terminal included in the user terminal group 110 is a terminal of a user who uses a training program according to the present disclosure, and refers to an electronic device equipped with a communication module capable of communicating with the management server 20.

The user terminal refers to a smart device including an input device for receiving an input from the user, an output device (e.g., a display) for visually outputting an input to the user terminal or a result of processing by the user terminal, and a communication module capable of communicating with an external device, and thus, the size or type of the user terminal is not limited as long as it includes the input device, the output device, and the communication module. For example, although FIG. 1 illustrates that the user terminal is a smart phone, the user terminal may be a personal computer (PC), a notebook computer, a netbook, or the like capable of communicating with the management server 20.

The user of the user terminal refers to a person who uses an application for improving symptoms of a patient with a cognitive impairment, and may be a patient diagnosed with a cognitive impairment or a guardian of the patient. As another example, the user may be a tester to improve the performance of the application by repeatedly executing the application.

The management server 20 is a server in which an integrated management program is installed, and refers to a server configured to manage and control a data flow while communicating with a plurality of user terminals included in the user terminal group 110. The integrated management program (e.g., an integrated management app) is installed in the management server 20, and according to an embodiment, a part of the integrated management program may be implemented in the form of a client to be executed by a user terminal, and installed in the user terminals included in the user terminal group 110.

The communication network 130 performs a function of connecting the user terminals included in the user terminal group 110 to the management server 20, and may include various wired and wireless communication networks such as a data network, a mobile communication network, or an Internet network.

In the present disclosure, the application refers to a logical device for controlling a screen output from the user terminal included in the user terminal group 110 under control of the management server 20. The application does not have a physical form and may be optimized for a user of the application multiple times. That is, the application may be continuously updated based on an input by the user through the user terminal and a command received from the management server 20. Patients with a cognitive impairment may improve symptoms of the cognitive impairment while repeatedly using the application running on the user terminals. The application is a program implemented with a process for improving symptoms of a cognitive impairment, and thus will be hereinafter referred to as a "training app" for convenience of description.

The training app may be installed in and executed by at least one of the user terminal and the management server 20. For example, the training app may be installed in the user terminal to perform control such that various training algorithms for improving symptoms of the user with a cognitive impairment are visually, aurally, and tactilely output. As another example, the training app may be implemented to be installed in the management server 20 to control content output from a display of the user terminal. In this case, only a simple client that visually, aurally, and tactilely outputs data received from the management server 20 is installed in the user terminal, and a function of controlling execution of a training algorithm flexibly according to an input from the user is performed by the training app installed in the management server 20. As another example, the training app may be implemented to be distributed and installed in the user terminal and the management server 20. That is, the scope of the present disclosure is not limited by any type of device in which the training app is installed or any type of installation of the training app, as long as the operation characteristics of the training app fall within the scope of the present disclosure.

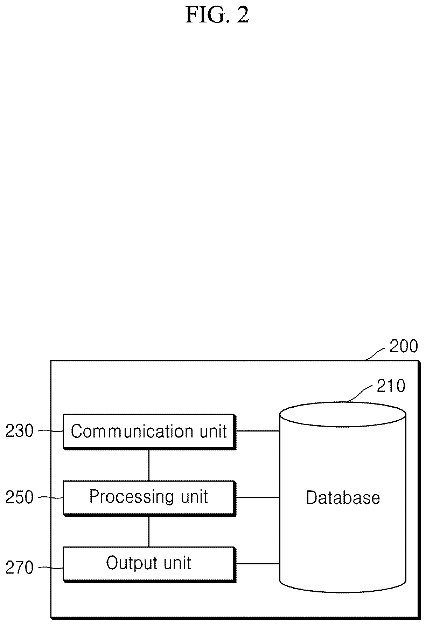

FIG. 2 is a block diagram illustrating a training app as a logical device, according to an embodiment of the present disclosure.

The training app is not physically sensed but is a logically tangible device for implementing a function of improving cognitive impairment symptoms according to the present disclosure, and may be hereinafter referred to as a "training device".

It may be seen from FIG. 2 that a training device 200 according to an embodiment of the present disclosure includes a database 210, a communication unit 230, a processing unit 250, and an output unit 270. According to an embodiment, the training device according to the present disclosure may be implemented in a form in which the communication unit 230 and the output unit 270 are included in the processing unit 250, and thus substantially operable only with the database 210 and the processing unit 250.

The database 210 stores various types of data necessary for the training device to operate. For example, the database 210 may store a program for executing a training algorithm for improving a cognitive function while communicating with the management server 20, and may perform a function of temporarily or semi-permanently storing data processed by the processing unit 250.

In a case in which the training device is installed in a user terminal, the communication unit 230 performs processing for communication with the management server 20. In detail, the communication unit 230 may perform an authentication procedure required to execute the training device while communicating with the management server 20. The authentication procedure may be performed with a prescription code issued by a doctor, which will be described below. In a case in which the training device is implemented in the management server 20, the communication unit 230 may be omitted.

The processing unit 250 processes data received by the communication unit 230 and data to be transmitted by the communication unit 230, and prepares and controls a screen output by the training device to the display of the user terminal. The processing unit 250 may refer to a data processing device having a structured circuitry to perform functions represented by code or instructions included in a program. For example, the processing unit 250 may be at least one of a microprocessor, a processor core, a multiprocessor, an application-specific integrated circuit (ASIC), and a fieldprogrammable gate array (FPGA). In a case in which the training device is not implemented as a physical device, but as only a logical device as described above, the processing unit 250 may be implemented in the form of a virtual machine that operates by imitating the operation characteristics of at least one of a microprocessor, a processor core, a multiprocessor, an ASIC, and an FPGA.

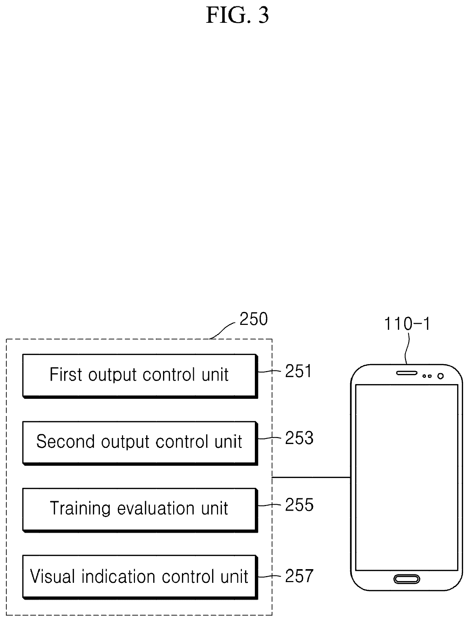

In an embodiment, the processing unit 250 may combine data received by the communication unit 230 with information stored in the database 210 and process a result of the combining, or may instruct the communication unit 230 and the output unit 270 to appropriately operate to implement a method according to the present disclosure. Functions performed by the processing unit 250 are not limited to a particular function, and although FIG. illustrates the processing unit 250 as a single module, the processing unit 250 may be divided into a plurality of submodules according to a process performed by the processing unit 250. The processing unit 250 consisting of submodules will be described below with reference to FIG. 3.

The output unit 270 receives a command from the processing unit 250 and generate and output various types of data. The output unit 270 receives a command from the processing unit 250 and generates and outputs various types of data, and according to an embodiment, may be implemented to be included in the processing unit 250 and thus omitted.

FIG. 3 is a block diagram illustrating an example of a processing unit subdivided by function.

In FIG. 3, a user terminal 110-1 is an example of one of n user terminals included in the user terminal group 110 described above with reference to FIG. 1, and refers to an electronic device having a display device for visually outputting a result of processing data by the user terminal 110-1, a speaker for aurally outputting the result, and an input device for receiving an input from a user.

Although FIG. 3 illustrates the user terminal 110-1 as a smart phone, the user terminal 110-1 in an implementation of the present disclosure is not limited to a smart phone, and may include a device installed in a fixed location, such as a general PC, or a portable terminal such as a tablet PC or a netbook. Accordingly, the training device 200 may be driven in a form included in other hardware devices such as a microprocessor or a general-purpose computer system. That is, the training device 200 may be mounted on the terminal 110-1 as it is, or may be implemented in a form that is connected to the terminal 110-1 through a wired/wireless network to control various pieces of data output to the display and the speaker of the terminal 110-1.

FIG. 3 mainly illustrates the processing unit 250 in the training device 200, and it may be seen from FIG. 3 that the processing unit 250 includes a first output control unit 251, a second output control unit 253, a training evaluation unit 255, and a visual indication control unit 257. In FIG. 3, the processing unit 250 and the submodules of the processing unit 250 are illustrated as components for emphasizing only the features of an embodiment of the present disclosure. Thus, those of skill in the art may understand that, according to an embodiment other than the embodiment illustrated in FIG. 3, other general-purpose components may be further included in addition to the components illustrated in FIG. 3.

The first output control unit 251 performs control such that a first training algorithm is output to a user terminal in response to an input to the user terminal. Here, the input to the user terminal means that a user applies a particular input to the user terminal through an input device, and may include not only an input of vowels and consonants for making a sentence, but also a touch input to a touchpad of the user terminal, a voice input to a speaker of the user terminal, a shake input by holding and shaking the user terminal, and the like. The shake input may be detected by motion sensors such as accelerometers or gyroscopes.

The user may perform training for improving symptoms of a cognitive impairment by checking a training algorithm output from the user terminal and applying an input corresponding to the training algorithm, and the first output control unit 251 may perform control such that the first training algorithm is output to the user terminal when an appropriate user input is detected. Here, the appropriate user input may be authentication information for outputting the first training algorithm. For example, the user may execute an app installed in the user terminal 110-1 and then log in, and in a case in which the user has not yet been granted permission to use the app, the user may perform a membership registration procedure through sign-up.

In an embodiment, the user may input a prescription code received from a doctor in addition to a normal log-in procedure, such that the first training algorithm is output. The first output control unit 251 may verify whether the input prescription code is a valid code through communication with an electronic medical record (EMR) server, and when the prescription code is valid, perform control such that the first training algorithm is output. The prescription code may be 8 digits, but is not limited thereto. According to an embodiment, the first output control unit 251 may process login information together with information authenticated with the prescription code.

In the present disclosure, the first training algorithm refers to a regular training algorithm that is first executed to improve symptoms of a cognitive impairment of the user, and is distinguished from a second training algorithm to be described below. The first training algorithm may include a plurality of detailed training algorithms, and accordingly, may be regarded as a kind of algorithm set. In some embodiments, the first output control unit 251 verifies the user and provides a training algorithm customized for the user as the first training algorithm.

When the first training algorithm is terminated, the second output control unit 253 may perform control such that the second training algorithm is output to the user terminal 110-1. Like the first training algorithm, the second training algorithm may include a plurality of detailed training algorithms, and accordingly may be regarded as a kind of algorithm set. The second output control unit 253 may receive history information about execution and termination of the first training algorithm by the first output control unit 251, and when there is valid history information, may perform control such that the second training algorithm is output to the user terminal 110-1. The detailed training algorithms included in the second training algorithm are different from the detailed training algorithms included in the first training algorithm, and the second training algorithm consists of algorithms for, in a case in which primary training for cognitive impairment symptoms of the user has been performed by the first training algorithm, greatly amplifying an effect of the primary training. That is, the second training algorithm refers to a training algorithm on the premise that the first training algorithm is preemptively performed.

In an embodiment, the second output control unit 253 may perform control such that the second training algorithm is output from the user terminal 110-1 after a preset time period elapses after the first training algorithm is terminated. An effect of improving cognitive impairment symptoms according to the present disclosure may be realized as the first training algorithm and the second training algorithm are sequentially and repeatedly performed, and in particular, in order to maximize a training effect of the second training algorithm, it is preferable that the user perform training according to the first training algorithm and then rest the brain for a preset time period. In order to maximize the training effect as described above, the second output control unit 253 may perform control such that the second training algorithm is output to the user terminal 110-1 only after a preset time period elapses, and until the preset time period elapses, may perform control such that the second training algorithm is not output even when the first training algorithm is terminated. Here, the preset time period may be 1 hour, and according to an embodiment, may be set to be shorter or longer than 1 hour.

In the present disclosure, the number of detailed training algorithms included in the first training algorithm may be a fixed first number. For example, the first number may be 3, but is not limited thereto.

In addition, in the present disclosure, the detailed training algorithms included in the second training algorithm may be a third number of detailed training algorithms selected from among a fixed second number of detailed training algorithm according to a predefined condition. For example, the second number may be 9 and the third number may be 4, but are not limited thereto. The preset condition set in the second output control unit 253 may vary.

In addition, in the present disclosure, a criterion for selecting the third number of detailed training algorithms may be a result of evaluating an input from the user. When there is a detailed training algorithm evaluated as a weakness in training by the user, the detailed training algorithm may be essentially included in training included in the third number of detailed training algorithms. The second output control unit 253 may identify the user's strengths and weaknesses in conjunction with the training evaluation unit 255, and induce training to proceed in a way of complementing the weaknesses. That is, the first training algorithm may be essentially all performed, but only some of the detailed training algorithms included in the second training algorithm may be performed, and a criterion for selecting some of the detailed training algorithms may be a training achievement level of the user.

The basic unit of the training for improving cognitive impairment symptoms according to the present disclosure may be one day. That is, the first training algorithm and the second training algorithm need to be sequentially performed on the same day to be effectively processed. For example, in a case in which the first training algorithm is terminated at 23:30 on a particular date and the preset time period required for outputting the second training algorithm is set to 1 hour, even when the user executes the training app at 0:30 on the next day after the first training algorithm is terminated, the second output control unit 253 may control the first output control unit 251 such that the first training algorithm for the first training of the day, not the second training algorithm, is output to the user terminal 110-1. A process of executing the first training algorithm and the second training algorithm on the same date will be described below.

When the second training algorithm is terminated, the training evaluation unit 255 may evaluate inputs from the user regarding the first training algorithm and the second training algorithm. Here, the inputs from the user regarding the first training algorithm and the second training algorithm may be a training result for the training algorithms. When the user terminal outputs the first training algorithm or the second training algorithm, at least some of inputs through the input device of the user terminal 110-1 may be training results for the first training algorithm or the second training algorithm, and the training evaluation unit 255 collects the training results to measure, as a grade or a score, whether the cognitive impairment symptoms of the user are improving or getting worse compared to the past.

The visual indication control unit 257 may perform control such that a first visual indication is output to the user terminal 110-1 based on a result of the evaluating by the training evaluation unit 255. Here, the first visual indication may be output through the display of the user terminal 110-1, and a visual effect (e.g., color, shape, pattern, or sparkle) of the first visual indication may vary depending on the user's diligence and performance for the training algorithms performed by the user.

For example, the first visual indication may be a flower of memory. In the present disclosure, the flower of memory is a representative example of a visual indication that may be variously displayed as the training for improving the cognitive impairment symptoms of the user is repeated, and improves in appearance as the user more diligently participates in the training and performs the training better.

The flower of memory may change in appearance from a bud to a flower in full bloom. In the present disclosure, the flower of memory does not simply provide an aesthetic effect to the user, but may show a better visual effect according to the user's training diligence or training performance, and thus motivate the user to participate in the training more diligently. That is, the flower of memory improves the sustainability of the training.

In addition, the visual indication control unit 257 may show the user a beautiful natural phenomenon, such as the flower of memory, in a repetitive and time-series manner, thereby visually stimulating an area in charge of cognitive function in the user's brain and inducing the cognitive impairment symptoms of the user to improve. In addition, the user may feel a sense of achievement while visually confirming the growing number of flowers of memory through the user terminal.

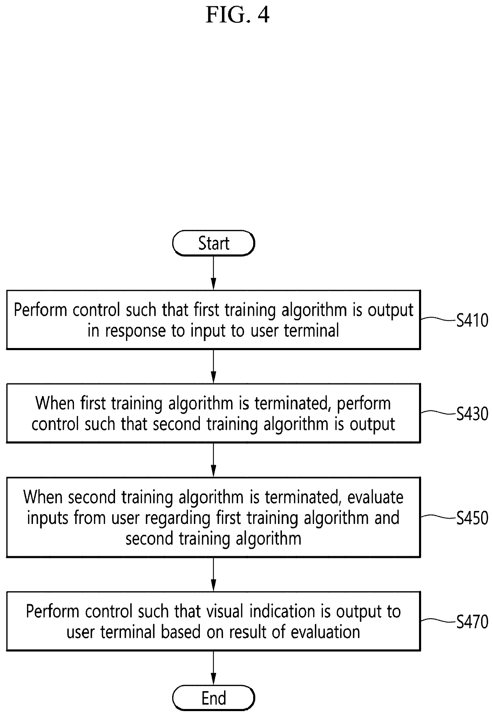

FIG. 4 is a flowchart illustrating an example of an operation process of submodules of the processing unit of FIG. 3.

The method of FIG. 4 may be performed by the first output control unit 251, the second output control unit 253, the training evaluation unit 255, and the visual indication control unit 257 described above with reference to FIG. 1, thus, hereinafter, descriptions will be provided with reference to FIG. 3, and the descriptions provided above with reference to FIG. 3 will be omitted.

The first output control unit 251 may perform control such that a first training algorithm is output in response to an input to a user terminal (S410).

When the first training algorithm is terminated, the second output control unit 253 may perform control such that a second training algorithm is output (S430).

When the second training algorithm is terminated, the training evaluation unit 255 may evaluate inputs from the user regarding the first training algorithm and the second training algorithm (S450).

The visual indication control unit 257 may perform control such that a visual indication is output to the user terminal based on a result of the evaluation performed by the training evaluation unit 255 (S470).

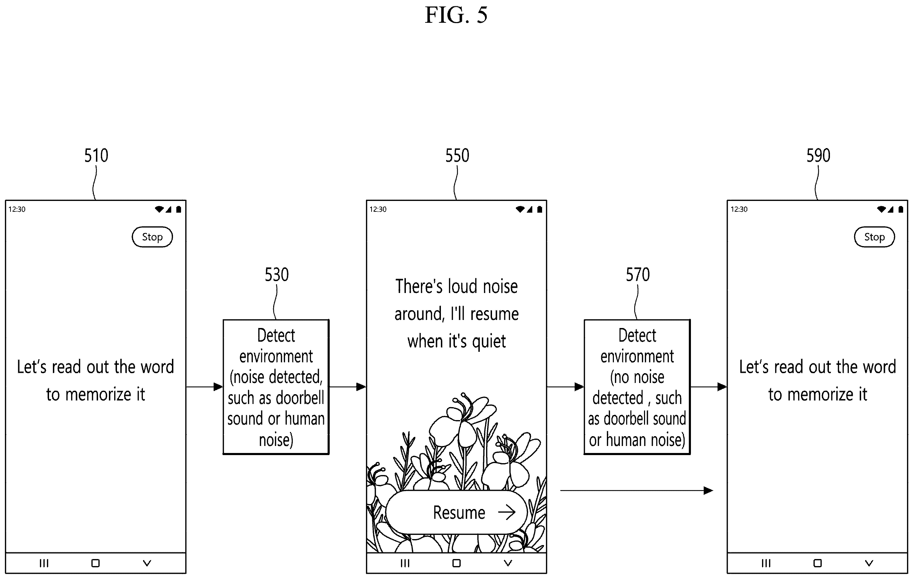

FIG. 5 is a diagram for describing a method of controlling an improved cognitive function training app, according to an embodiment of the present disclosure.

It is described above with reference to FIG. 3 that the processing unit 250 may include the first output control unit 251, the second output control unit 253, the training evaluation unit 255, and the visual indication control unit 257, and the first output control unit 251, the second output control unit 253, the training evaluation unit 255, and the visual indication control unit 257 may logically implement an application for training the user's cognitive function (hereinafter, referred to as a "cognitive function training app") by controlling a screen output to the user terminal 110-1.

Hereinafter, a method of controlling an improved cognitive function training app for significantly improving an effect of cognitive function training provided to a user by effectively controlling the above-described process performed by the cognitive function training app will be described.

**[Table 1]**

| Reference numeral | Name of module | Main function of module |
|---|---|---|
| 259 | Control system | Implement a method of controlling an improved cognitive function training app |
| 2591 | Initiation determination unit | Determine whether the cognitive function training app has been initiated |
| 2593 | Training optimization unit | Optimize a process of cognitive function training to be appropriate for the current state of a user |
| 2595 | Training provision unit | Perform control such that the optimized cognitive function training may be provided to the user |

Table 1 is for describing a device for implementing the above-described method of controlling an improved cognitive function training app. In more detail, the modules in Table 1 are not directly illustrated in FIG. 3, but refer to detailed modules of devices for implementing the above-described method of controlling an improved cognitive function training app, and may be implemented in a form physically or logically included in the processing unit 250.

A control system 259 is a system that physically or logically includes modules for implementing the method of controlling an improved cognitive function training app according to an embodiment of the present disclosure, and may include an initiation determination unit 2591, a training optimization unit 2593, and a training provision unit 2595.

The initiation determination unit 2591 may determine whether the cognitive function training app has been initiated. In more detail, the initiation determination unit 2591 may determine whether the cognitive function training app has been initiated by an input from the user to the user terminal 110-1, and deliver a result of the determining to the training optimization unit 2593.

For example, in a case in which the user successfully executes the cognitive function training app installed in the user terminal 110-1 by using various input devices (e.g., a microphone, a touch pad, or a keyboard) provided in the user terminal 110-1, the initiation determination unit 2591 may determine that the cognitive function training app has been initiated. As another example, in a case in which user B executes the cognitive function training app installed in the user terminal 110-1 owned by user A, and logs in as user B by inputting account information (e.g., an ID and a password) of user B, the initiation determination unit 2591 may determine that the cognitive function training app has not been normally initiated. That is, only in a case in which the cognitive function training app is normally executed by the terminal owned by the user and with personal information of the user, the initiation determination unit 2591 may determine that the cognitive function training app has been initiated. The initiation determination unit 2591 may utilize not only login information of the user but also other information such as fingerprint information of the user, in order to perform the above authentication procedure.

Subsequently, the training optimization unit 2593 may optimize, for the user, cognitive function training provided by the cognitive function training app, based on a result of the determining of whether the cognitive function training app has been initiated and information of the user of the user terminal 110-1.

Here, the training optimization unit 2593 may identify whether the cognitive function training app has been initiated, by receiving information from the initiation determination unit 2591. In addition, the information of the user of the user terminal 110-1 determined by the training optimization unit 2593 may be information previously stored in the user terminal 110-1, and as another example, may be information of the user that is input by the user to the user terminal 110-1 within a preset time period after the training optimization unit 2593 starts optimization of the cognitive function training app. The training optimization unit 2593 may receive, from the user, consent to collection of personal information in order to obtain information previously stored in the user terminal 110-1 as the information of the user.

In an embodiment, the information of the user of the user terminal 110-1 may be information previously collected by a sensor of the user terminal 110-1. Here, the sensor includes not only a sensor inseparably coupled to the user terminal 110-1, but also a sensor detachable from the user terminal 110-1, and thus may be one or more of a microphone, a camera, a proximity sensor, an illuminance sensor, a temperature sensor, a humidity sensor, a pressure sensor, a global positioning system (GPS) sensor, an acceleration sensor, a gravity sensor, a rotation sensor, a geomagnetic sensor, a distance sensor, a fingerprint sensor, an iris sensor, and a heartbeat sensor (a kind of optical sensor). In addition, information about a biosignal-based circadian rhythm of the user collected by the sensor of the user terminal 110-1, and a schedule of the user may also be the information of the user obtained by the training optimization unit 2593.

In another embodiment, the information of the user of the user terminal 110-1 may be information that is input after the cognitive function training app is first executed. For example, when the cognitive function training app is installed in and executed by the user terminal 110-1, the cognitive function training app may collect information about at least one of the sleeping time, the number of housemates, the outdoor activity level, and the depression level of the user by using a questionnaire, and the collected information may be used by the training optimization unit 2593 with the consent of the user.

Here, the sleeping time and the number of housemates of the user may be values selected from integers within a preset range. In addition, the outdoor activity level of the user may be a value representing the user's subjective feeling, and for example, level 5 may be a value selected by a user who does a lot of outdoor activities, and level 1 may be a value selected by a user who does indoor activities alone.

Like the outdoor activity level, the depression level may be a value representing the user's subjective feeling, and according to an embodiment, may be an evaluation value obtained through the Beck Depression Inventory or a value representing a Patient Health Questionnaire-9 (PHQ-9) score. The above-described information about the outdoor activity level and the depression level is an example, and thus, in the present disclosure, is not limited to a particular method.

In another embodiment, the information of the user of the user terminal 110-1 may be external information about the day on which the cognitive function training app provides cognitive function training, and the user's personal information about the day. Here, examples of external information about the day of providing the cognitive function training may include information about the weather and temperature on the day on which the cognitive function training is performed.

In the present embodiment, the user's personal information may be information about the day, and when the user's personal information is information in the form of a photo, memo, or message, the personal information may be information collected 1 to 2 weeks prior to the day, and when the user's personal information is information about a schedule, the personal information may be information about schedules for a period 1 to 2 weeks prior to the day and a period 1 to 2 weeks subsequent to the day.

In addition, the personal information of the user of the user terminal 110-1 may include at least one of Short Messaging Service (SMS)- or Multimedia Messaging Service (MMS)-type messages, search words, conversations on messengers, a YouTube watch history, and national medical records stored in the user terminal.

That the training optimization unit 2593 optimizes the cognitive function training app means that the training optimization unit 2593 adjusts a process of the cognitive function training app most appropriately for the user who logs in through a legal procedure and receives the cognitive function training through the user terminal 110-1, and when the cognitive function training app is optimized, the cognitive function training may be provided to the user with higher efficiency, and the sustainability may be improved as the user has fun while participating in the cognitive function training. For example, the control system 259 may include a default cognitive function training app to be provided to users in general. The training optimization unit 2593 may adjust the default cognitive function training app to an updated cognitive function training app customized for the user based on inputs from the user and/or information about the user previously registered to the control system 259.

Here, adjusting the process of the cognitive function training app means changing the format in which the cognitive function training app provides the cognitive function training to the user, within a certain range. For example, when a user with significantly reduced cognitive function may feel that the speed of the cognitive function training provided by the cognitive function training app is too fast, the method according to an embodiment of the present disclosure may help the user recover from a cognitive impairment through the cognitive function training, by lowering the speed of the cognitive function training to a certain level. In addition, according to the present disclosure, when the external situation surrounding the user rapidly changes and the user cannot normally perform the cognitive function training even after the user has initiated the cognitive function training, the initiated cognitive function training may be controlled to first pause and then resume when a preset time period has elapsed and the external situation surrounding the user has improved. In addition, when the user's health condition rapidly deteriorates or the user's fatigue level is excessive, the sensor activated in the user terminal 110-1 may detect the deterioration of the user's health condition or high fatigue level such that the training optimization unit 2593 may stop (determine whether to proceed with) the cognitive function training according to the current state of the user or appropriately delay the speed of the cognitive function training.

In the present disclosure, the optimization of the cognitive function training may mean changing the process of the cognitive function training in which one cycle is repeatedly performed, according to the user. For example, when a cognitive function training process having a duration of exact 10 minutes is initiated, then proceeds for 4 minutes, then is stopped for 5 minutes due to the need for optimization, and then resumes to be completed, the cognitive function training with one 10-minute cycle is recorded as having been completed within 15 minutes, and such an increase in cycle length may be an example of the optimization in the present disclosure.

As another example, in the present disclosure, the optimization of the cognitive function training may mean changing a process of the cognitive function training by using arbitrary visual or auditory data according to the user. For example, while the process of the cognitive function training is in progress, an image or music that may increase the user's concentration or attention may be provided to the user through the user terminal 110-1, and such insertion of images and music is only for a particular user, and thus may be seen as optimization for the user.

**[Table 2]**

| |
|---|
| Training 1 has begun. The words for today's training are 'street light' and 'umbrella'. |
| It's raining all day today, and amazingly, these words are provided, making the word training easier. Today, I qot a picture of my grandson with a yellow umbrella and |
| raincoat from the kindergarten, and it was so cute. I love that the Cogthera app seems to know my day and qives me words related to myself so I can easily make a story. |

**[Table 3]**

| |
|---|
| After figuring out my condition, Laura plays fast-beat music. She also provides eyerefreshing images and videos. The app provides different wallpapers depending on the day's training time. The app provides different background colors for breakfast, lunch, afternoon, evening, and weather so I feel like the app is alive, and I can more concentrate on the screen and make a store better. Green increases concentration and yellow increases vitality. The Cogthera app provides various background colors/images/videos and fonts, and various shapes and sizes of fonts are presented. |
| Perhaps because of these things, it seems that imagination and concentration increase, so it is fun to train. |

Tables 2 and 3 exemplarily show monologues of particular users. In Tables 2 and 3, the Cogthera app is regarded as an example of a cognitive function training app.

In more detail, Table 2 shows a result obtained through a process in which the training optimization unit 2593 extracts images or texts from the weather, photos stored in the user terminal 110-1, memos stored in the user terminal 110-1, and schedules stored in the user terminal 110-1, optimizes the cognitive function training based on the extracted images and texts, and provides the user with the optimized cognitive function training, and the user may perform training to effectively improve his/her cognitive function through familiar images or texts.

In addition, Table 3 exemplarily shows that the user effectively performs cognitive function training with fast-beat music or with the help of colors or images that may improve concentration.

When the cognitive function training app is initiated or resumed by the user, the training provision unit 2595 may provide the user with the optimized cognitive function training through the user terminal 110-1. That is, when an optimized component only for the user determined by the training optimization unit 2593 is reflected in the cognitive function training, the training provision unit 2595 executes the cognitive function training in which the optimized component is reflected to provides it to the user, and such a function of the training provision unit 2595 may be differentiated from the function of a module that executes the cognitive function training previously stored in the database 210 as it is without modification.

Hereinafter, functions of the control system 259 and the submodules included in the control system 259 described above will be described with reference to FIG. 5.

A first screen 510 of FIG. 5 is an example of a screen that may be output when the cognitive function training app is executed by the user terminal 110-1. In detail, the first screen 510 of FIG. 5 requests the user to "read out the word to memorize it", and after a preset time period has elapsed, the word to be pronounced by the user may be output.

The training optimization unit 2593 may detect that a sound irrelevant to the user's voice is input through the microphone of the user terminal 110-1 in excess of a preset volume (i.e., a preset level), temporarily stop the cognitive function training, and optimize the cognitive function training to provide the user with visual guide information and auditory guide information 530. For example, the microphone of the user terminal 110-1 records audio data including the combination of the audio of the user and a sound irrelevant to the user's voice. The processing unit of the user terminal 110-1 implements audio processing on the audio data to extract the sound irrelevant to the user's voice out of the audio data. The processing unit of the user terminal 110-1 may extract voice characteristics of the audio of the user such as a frequency, tone, volume and the like, and extract the sound irrelevant to the user's voice by filtering out the audio of the user based on the voice characteristics of the audio of the user. Then, the processing unit of the user terminal 110-1 determines whether the volume of the sound irrelevant to the user's voice is greater than the preset level. If the volume of the sound irrelevant to the user's voice is greater than the present level, the processing unit of the user terminal temporality stops the cognitive function training. A second screen 550 of FIG. 5 schematically shows that visual guide information is provided to the user. When a preset time period has elapsed or the user actively clicks a "Resume" button after the cognitive function training is temporarily stopped, the cognitive function training may be resumed 570, and a third screen 590 of FIG. 5 schematically shows that the cognitive function training is resumed.

The sound irrelevant to the user's voice may be a sound of pouring water into a cup, noise from a sink or a toilet in a bathroom, a phone ringtone, a voice of a person other than the user, a television (TV) sound, an announcement, a sound from a siren, a sound louder than living noise, or the like.

In an alternative embodiment, when a sound irrelevant to the user's voice is input in excess of the preset volume, but the user's voice is clearly input, the training optimization unit 2593 may not perform optimization of the cognitive function training in order to prevent delay in the training and ensure user convenience. In this case, the training optimization unit 2593 may additionally request consent of the user to the omission of optimization of the cognitive function training.

In some embodiments, the control system 259 may obtain the current location of the user terminal 110-1 and determines whether or not temporarily stop the cognitive function training based on the location of the user terminal 110-1. For example, if the current location of the user terminal 110-1 does not match with one of the primary locations of the user such as home addresses, frequency visiting locations including hospitals, nursery and the control system 259 detects that a sound irrelevant to the user's voice is input through the microphone of the user terminal 110-1 in excess of a preset volume, the training optimization unit 2593 may temporarily stop the cognitive function training and output instructions for the user to move to one of the primary locations and resume the cognitive function training.

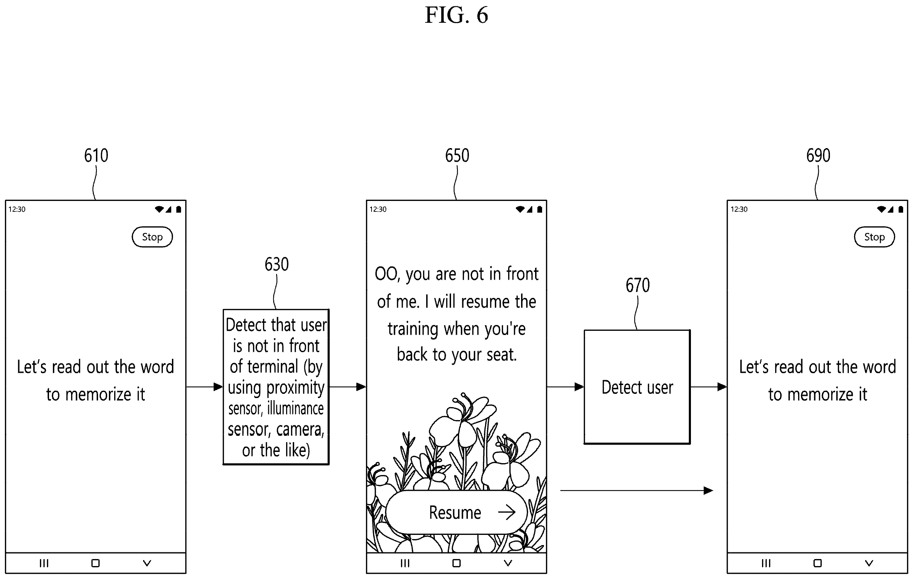

FIG. 6 is a diagram for describing an embodiment in which cognitive function training is optimized as the absence of a user is detected.

A first screen 610 of FIG. 6 shows that the cognitive function training app is normally initiated by the user. Here, in a situation in which the sensor of the user terminal 110-1 detects that the user is not around the user terminal 110-1 in step 630 of FIG. 6, and thus, the cognitive function training cannot be normally performed, the training optimization unit 2593 may output a second screen 650 of FIG. 6 to the user terminal 110-1 and temporarily stop the cognitive function training. For example, the proximity sensor of the user terminal 110-1 may detect an object in front of the user terminal 110-1 and the camera of the user terminal 110-1 may capture the image of the object and process the captured image to identify the user. As another example, the illuminance sensor of the user terminal 110-1 may detect illuminance of the ambience of the user terminal 110-1 and determine whether the user is present. When the user is detected in step 670, output a third screen 690 of FIG. 6 to resume the cognitive function training.

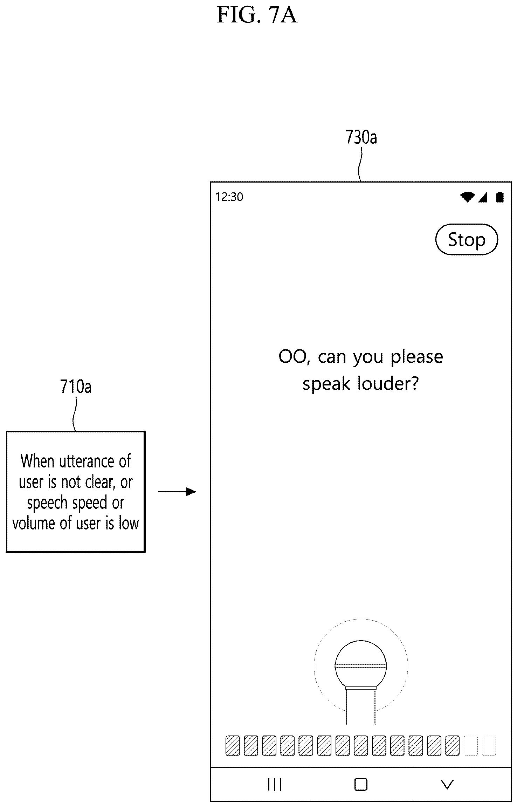

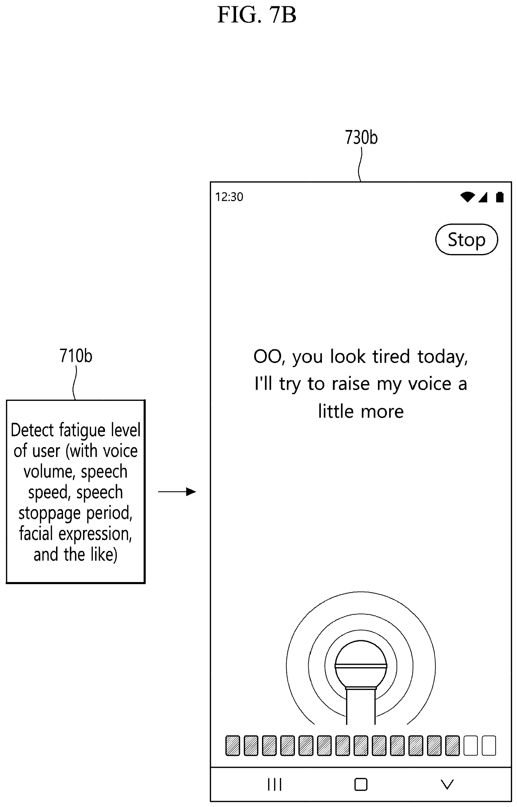

FIGS. 7A and 7B are diagrams schematically illustrating examples in which cognitive function training is optimized in a situation in which a user's voice is normally input.

First, FIG. 7A is a diagram schematically illustrating a situation in which, when it is recognized, by analyzing a voice of the user collected by the sensor of the user terminal 110-1, that an utterance of the user is not clear, the speech speed of the user is slow, or the volume of an utterance of the user is low 710a, the cognitive function training is temporarily delayed rather than immediately performing the cognitive function training, and the user is induced to speak again. For example, the microphone of the user terminal 110-1 may collect voice data of the user and convert the voice data to a text using a speech recognition algorithm. The control system 259 of the user terminal 110-1 may not be able to convert all voice data into a text if the utterance of the user is not clear. As another example, the microphone of the user terminal 110-1 may collect voice data of the user and convert the voice data to a text using a speech recognition algorithm. The control system 259 of the user terminal 110-1 may calculate the speech speed of the user, for example, the number of words or characters spoken per minute based on the converted text. In this process, after determining that the cognitive function training app has been initiated, the training optimization unit 2593 optimizes the cognitive function training in real time by considering collected inputs of the user (in a case in which the inputs from the user exceed or fall short of a preset level), and this situation may occur even when there is no input irrelevant to the user, unlike the example illustrated in FIG. 5. The user may visually check a guide screen 730a of FIG. 7A or check auditory guide information output through the user terminal 110-1, and then speak again to resume the cognitive function training.

Next, FIG. 7B is a diagram 730b schematically illustrating that the training optimization unit 2593 calculates the user's fatigue level and optimizes cognitive function training based on the calculated fatigue level 710b. The situation illustrated in FIG. 7B may be valid in the following situation.

**[Table 4]**

| |
|---|
| Maybe because I overworked today for making Kimchi, my eyes were slowly closing when the second training began. I couldn't hear Laura's question, so I stopped and restarted the training twice. An announcement suddenly comes from the apartment |
| security room. The Cogthera app hears the sound and pauses the training. After the announcement is over, I press the start button on the screen to resume the training. |
| My brain doesn't work well because I'm tired. When I respond to the training instructions, I unknowingly stay in a daze and start to answer slowly. Speech becomes slower, and the voice becomes quieter. |

Table 4 shows a monologue of a 68-year-old female user, and exemplarily depicts a situation in which a user using the cognitive function training app feels a high fatigue level. The training optimization unit 2593 may collect the user's voice volume, speech start timing, speech speed, speech habit, blink rate, blink speed, and pupil size, through various sensors (e.g., a microphone, a camera, a timer, or an iris sensor) activated in the user terminal 110-1, calculate the user's fatigue level in real time by considering at least one of them, and optimize the cognitive function training such that a process of the cognitive function training is stopped or delayed when the calculated fatigue level exceeds a preset level. For example, when the voice volume is low, the speech start timing is late, the speech speed is slow, or the blink rate is significantly reduced, the training optimization unit 2593 may determine that the user's fatigue level is higher than usual.

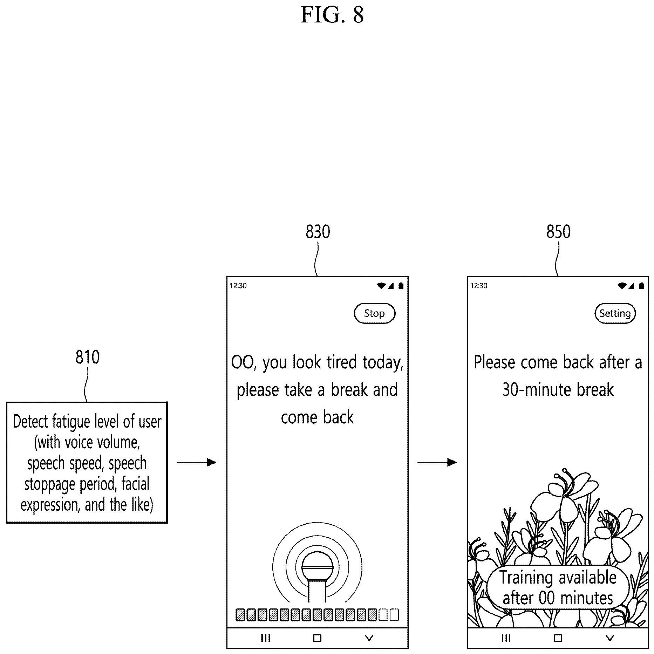

FIG. 8 is a diagram exemplarily illustrating a situation in which cognitive function training is stopped.

A third screen 850 of FIG. 8 is a screen that is output through the user terminal 110-1 when it is determined that the cognitive function training cannot proceed normally in the same situation 810 as in FIGS. 7A and 7B, and the training optimization unit 2593 and the training provision unit 2595 may stop the cognitive function training app 830 for a preset time period to allow the user to take a sufficient break, and then resume the cognitive function training app.

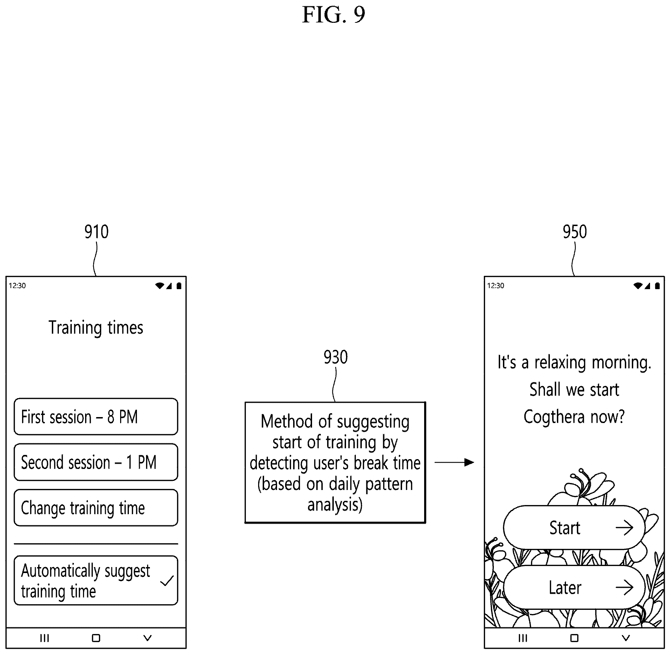

FIG. 9 is a diagram schematically illustrating an example of outputting a training time range most appropriate for a user.

The user may execute the cognitive function training app and set a training time appropriate for the user, and as in a training time setting screen 910 of FIG. 9, the user may set the cognitive function training app to automatically suggest a training time. In a state in which the cognitive function training app is not initiated, the training optimization unit 2593 may visually or audibly output a training time range most appropriate for the cognitive function training to the user through the user terminal 110-1, based on a result of performing pattern recognition and analysis on the daily life of the user collected by the sensor of the user terminal 110-1. A training time recommendation screen 950 of FIG. 9 schematically illustrates an embodiment in which training is suggested as the user terminal 110-1 is activated regardless of an input from the user in a state in which a training time is determined by the training optimization unit 2593. The training optimization unit 2593 may optimize one-cycle cognitive function training to include, at the beginning of the cognitive function training, a process of calculating a time range of day most appropriate for performing the cognitive function training by generally considering information such as the user's circadian rhythm and schedule, and suggesting the calculated time range to the user.

**[Table 5]**

| |
|---|
| When registering the app, I can input my sleeping time, the number of family members living with me, my outdoor activity level and physical exercise level, and my depression level. The Cogthera app identifies my sleeping time, circadian rhythm, and schedule to suggest the optimal time to enhance my cognitive training. It is said that the concentration of normal people rises in the morning, slightly drops at lunch, and rises again in the evening. People slightly differ from each other in concentration, and the Cogthera app suggests that my brain is most active between 7 PM and 9 PM. |
| When I runs the app at about 8 PM, a greeting is output saying "Now is the training time to get the highest score". I feel good because the app seems to know me well. It was a tiring day, but I think today's training will go well. |

Table 5 is an example of a user's impression for describing the training time recommendation process described above with reference to FIG. 9. As shown in Table 5, the user may be provided with a recommendation for the most appropriate training time for the user, and perform training at the recommended time to gain high achievement. The recommended training time may be a time point at which the user is least busy considering the user's schedule, and the user's circadian rhythm is most stable.

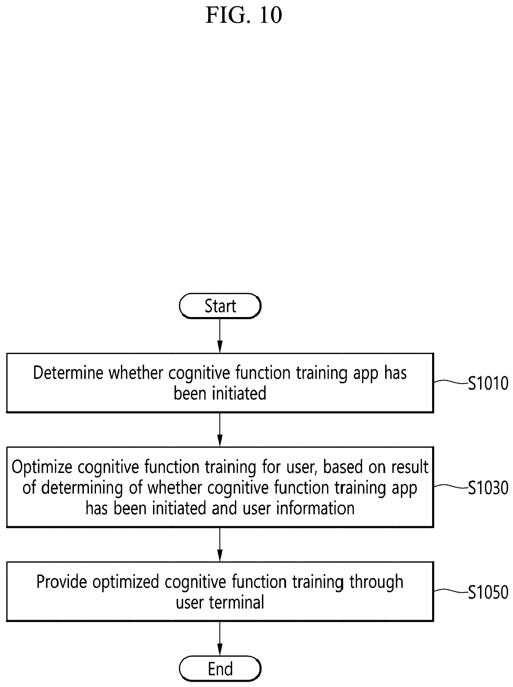

FIG. 10 is a flowchart illustrating an example of a method of controlling the cognitive function training app described above with reference to FIGS. 5 to 9.

The method of FIG. 10 may be performed by the control system 259 and the submodules of the control system 259 described above with reference to FIG. 5, and thus, the descriptions provided above with reference to FIGS. 5 to 9 will be omitted.

The initiation determination unit 2591 may determine whether the cognitive function training app has been initiated by an input from the user to the user terminal 110-1 (S1010).

The training optimization unit 2593 may optimize, for the user (for the environment and state of the user), cognitive function training provided by the cognitive function training app, based on a result of the determining of whether the cognitive function training app has been initiated and information of the user of the user terminal 110-1 (S1030).

When the cognitive function training app is initiated or resumed by the user, the training provision unit 2595 may provide the optimized cognitive function training through the user terminal 110-1 (S1050).

In embodiments, the processing unit of the user terminal 110-1 determines whether there is cognitive function decline based on inputs from the user regarding the optimized cognitive function training. Specifically, if it is determined that there is cognitive function decline in a neurocognitive category, the processing unit may perform actions for treating the user. For example, the processing unit may search hospitals that may treat the cognitive function decline based on the location of the user. The location of the user may be determined based on the location of device of the user, such as the user terminal 110-1 in FIG. 1. The location of the device of the user may be obtained, e.g., by receiving a GPS signal from the user terminal 110-1. Then, the processing unit may transmit information about the searched hospitals to the user terminal 110-1 such that the screen of the user terminal 110-1 displays the information about the searched hospitals such as locations of the hospitals, doctors at the hospitals, available treatments, available medical devices, and the like. The processing unit may reserve an appointment with a doctor at one of the hospitals for the user and transmit information about the appointment to the doctor.

As another example, if it is determined that there is cognitive function decline in a neurocognitive category, the processing unit may determine a suggested test, such as, an MRI exam, a CT exam, a PET exam, a blood test, a Cerebrospinal fluid test based on the test result, and transmit the suggested test to the user terminal 110-1 such that the screen of the user terminal 110-1 displays the information about the suggested test. The information about the suggested test may be transmitted to a primary doctor of the user.

As another example, if it is determined that there is cognitive function decline in a neurocognitive category, the processing unit may provide additional questions to the user, such as questions regarding physical Activity of daily living and instrumental activity of daily living, questions for determining whether the cognitive function decline is due to senile depression, questions for confirming behavioral and psychological Symptom in Dementia.

In embodiments, if it is determined that the user may have a certain disease or a dementia based on the test results, the processing unit may formulate additional tests customized for the user. For example, if the user is suspected to have Parkinson disease, Parkinson disease dementia, or Lewy body dementia, the processing unit may perform a color vision test to check the user's ability to distinguish between colors. As another example, if the user is suspected to have frontotemporal dementia, the processing unit may perform additional tests such as a theory of mind test, a facial emotion test, and the like, in order to more accurately evaluate the level of social cognition of the user.

According to the present disclosure, it is possible to provide a training algorithm for dramatically improving cognitive impairment symptoms of a patient with a cognitive impairment, in various forms.

The embodiments of the present disclosure described above may be implemented as a computer program that may be executed through various components on a computer, and such a computer program may be recorded in a computer-readable medium. In this case, the medium may include a magnetic medium, such as a hard disk, a floppy disk, or a magnetic tape, an optical recording medium, such as a compact disc read-only memory (CD-ROM) or a digital video disc (DVD), a magneto-optical medium, such as a floptical disk, and a hardware device specially configured to store and execute program instructions, such as ROM, RAM, or flash memory.

Meanwhile, the computer program may be specially designed and configured for the present disclosure or may be well-known to and usable by those skilled in the art of computer software. Examples of the computer program may include not only machine code, such as code made by a compiler, but also high-level language code that is executable by a computer by using an interpreter or the like.

Particular executions described herein are merely examples and do not limit the scope of the present disclosure in any way. For the sake of brevity, conventional electronics, control systems, software and other functional aspects of the systems may not be described in detail. Furthermore, line connections or connection members between elements depicted in the drawings represent functional connections and/or physical or circuit connections by way of example, and in actual applications, they may be replaced or embodied with various suitable additional functional connections, physical connections, or circuit connections. In addition, no item or component is essential to the practice of the present disclosure unless the item or component is specifically described as being "essential" or "critical".

The term 'the' and other demonstratives similar thereto in the specification of the present disclosure (especially in the following claims) should be understood to include a singular form and plural forms. Furthermore, recitation of ranges of values herein are merely intended to serve as a shorthand method of referring individually to each separate value falling within the range, unless otherwise indicated herein, and each separate value is incorporated into the specification as if it were individually recited herein. Finally, the operations of the methods described herein may be performed in any suitable order unless otherwise indicated herein or otherwise clearly contradicted by context. The present disclosure is not limited to the described order of the operations. The use of any and all examples, or exemplary language (e.g., 'and the like') provided herein, is intended merely to better illuminate the present disclosure and does not pose a limitation on the scope of the present disclosure unless otherwise claimed. In addition, various modifications, combinations, and adaptations will be readily apparent to those skill in the art without departing from the following claims and equivalents thereof.

According to the present disclosure, a symptom of a user suffering from a cognitive impairment may be remarkably improved.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. A method of controlling an improved cognitive function training app, the method comprising:
determining whether the cognitive function training app has been initiated by an input from a user to a terminal;
providing cognitive function training of the cognitive function training app through an output device of the terminal based on a result of the determining of whether the cognitive function training app has been initiated;
optimizing the cognitive function training by changing a format in which the cognitive function training app provides the cognitive function training based on information of the user of the terminal; and
based on the cognitive function training app being initiated or resumed by an input from the user, providing the optimized cognitive function training through the terminal.

2. The method of claim 1, wherein the information of the user is information that is previously collected by a sensor of the terminal.

3. The method of claim 1, wherein the information of the user is information about a biosignal-based circadian rhythm of the user collected by a sensor of the terminal, and a schedule of the user.

4. The method of claim 1, wherein the information of the user is at least one of a sleeping time of the user, a number of housemates of the user, an outdoor activity level of the user, and a depression level of the user, which are input after the cognitive function training app is first executed.

5. The method of claim 1, wherein the optimizing comprises visually or aurally outputting a training time range of day that is most appropriate for the user.

6. The method of claim 1, wherein the information of the user is external information about a day on which the cognitive function training app provides the cognitive function training, and personal information of the user on the day.

7. The method of claim 1, wherein the optimizing comprises, after determining that the cognitive function training app has been initiated, optimizing the cognitive function training in real time by considering collected inputs from the user.

8. The method of claim 7, wherein the optimizing further comprises, based on the inputs from the user being greater than a preset level, stopping the cognitive function training for a preset time period, or changing a training speed of the cognitive function training.

9. The method of claim 7, wherein the optimizing further comprises, based on the inputs from the user being less than or equal to a preset level, stopping the cognitive function training for a preset time period, or changing a training speed of the cognitive function training.

10. The method of claim 7, wherein the optimizing further comprises collecting inputs irrelevant to the user in addition to the inputs from the user, and based on the collected inputs being greater than a preset level, stopping the cognitive function training.

11. The method of claim 10, wherein the providing of the optimized cognitive function training through the terminal comprises stopping the cognitive function training until the collected inputs fall below a certain level.

12. The method of claim 1, wherein the providing of the optimized cognitive function training through the terminal comprises, based on the input from the user satisfying a preset condition, outputting at least one of visual guide information and auditory guide information through the terminal.

13. The method of claim 1, wherein the optimizing comprises calculating a fatigue level of the user based on a new input from the user, and adjusting a progress speed of the optimized cognitive function training based on the calculated fatigue level.

14. The method of claim 1, wherein the optimizing comprises calculating a fatigue level of the user based on the input from the user, and determining whether to proceed with the optimized cognitive function training, based on the calculated fatigue level.

15. A device for controlling an improved cognitive function training app, the device comprising:
a processor programmed to:
determine whether the cognitive function training app has been initiated by an input from a user to a terminal;
provide cognitive function training of the cognitive function training app through an output device of the terminal based on a result of the determining of whether the cognitive function training app has been initiated;
optimize the cognitive function training by changing a format in which the cognitive function training app provides the cognitive function training based on information of the user of the terminal; and
based on the cognitive function training app being initiated or resumed by an input from the user, provide the optimized cognitive function training through the terminal.
